(19) Europäisches Patentamt
European Patent Office
Office européen des brevets

(11) Publication number: **0 349 956 A1**

(12) # EUROPEAN PATENT APPLICATION

(21) Application number: 89112122.0

(51) Int. Cl.4: C07D 417/04 , A61K 31/44

(22) Date of filing: 03.07.89

(30) Priority: 04.07.88 DK 3710/88

(43) Date of publication of application:
10.01.90 Bulletin 90/02

(84) Designated Contracting States:
AT BE CH DE ES FR GB GR IT LI LU NL SE

(71) Applicant: A/S FERROSAN
Sydmarken 5
DK-2860 Soeborg(DK)

(72) Inventor: Sauerberg, Per
Sondervangs Alle 56B
DK-2500 Valby(DK)

(74) Representative: Patentanwälte Grünecker, Kinkeldey, Stockmair & Partner
Maximilianstrasse 58
D-8000 München 22(DE)

(54) **Azacyclic compounds and their preparation und use.**

(57) Novel azacyclic compounds having the formula

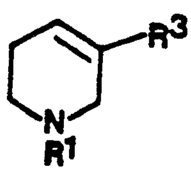

wherein
$R^1$ is H or $C_{1-6}$-alkyl, and

$R^3$ is 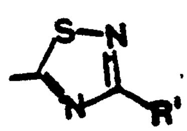

wherein $R'$ is hydrogen, $C_{1-6}$-alkyl, $C_{3-8}$-cycloalkyl, $C_{1-4}$-alkoxy, $C_{1-6}$-alkenyl, $C_{1-6}$-alkynyl, halogen or amino.

The new compounds are useful in improving the cognitive functions of the forebrain and hippocampus of mammals, and are useful in the treatment of Alzheimer's disease.

EP 0 349 956 A1

## Azacyclic Compounds and Their Preparation and Use

The present invention relates to therapeutically active azacyclic compounds, a method of preparing the same and to pharmaceutical compositions comprising the compounds. The novel compounds are useful as stimulants of the cognitive function of the forebrain and hippocampus of mammals and especially in the treatment of Alzheimer's disease.

Due to the in general improved health situation in the western world, elderly-related diseases are much more common now than in the past and are likely to be even more common in the future.

One of the elderly-related symptoms is a reduction of the cognitive functions. This symptom is especially pronounced in the patophysiological disease known as Alzheimer's disease. This disease is combined with, and also most likely caused by, a up to 90% degeneration of the muscarinic cholinergic neurons in nucleus basalis, which is part of substantia innominata. These neurons project to the prefrontal cortex and hippocampus and have a general stimulatory effect on the cognitive functions of the forebrain as well as of hippocampus, namely learning, association, consolidation, and recognition.

It is a characteristic of Alzheimer's disease that although the cholinergic neurons degenerate, then the postsynaptic muscarinic receptors in the forebrain and hippocampus still exist. Therefore muscarinic cholinergic agonists are useful in the treatment of Alzheimer's disease and in improving the cognitive functions of elderly people.

It is well known that arecoline (methyl 1-methyl-1,2,5,6-tetrahydropyridine-3-carboxylate) is such a cholinergic agonist.

Arecoline however has a very short biological half life and a small separation between central and peripheral muscarinic effects. Furthermore arecoline is a rather toxic compound.

Accordingly it is an object of the invention to provide new muscarinic cholinergic compounds.

The novel compounds of the invention have the general formula I:

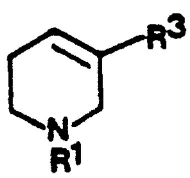

(I)

wherein
$R^1$ is H or $C_{1-6}$-alkyl, and

$R^3$ is 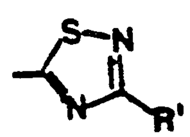

wherein $R'$ is hydrogen, $C_{1-6}$-alkyl, $C_{3-8}$-cycloalkyl, $C_{1-4}$-alkoxy, $C_{1-6}$-alkenyl, $C_{1-6}$ -alkynyl, halogen or amino.

Examples of such salts include inorganic and organic acid addition salts such as hydrochloride, hydrobromide, sulphate, phosphate, acetate, fumarate, maleate, citrate, lactate, tartrate, oxalate, or similar pharmaceutically-acceptable inorganic or organic acid addition salt.

The invention also relates to a method of preparing the above mentioned compounds. This method comprises

a) reacting a reactive derivative of a compound having the formula II

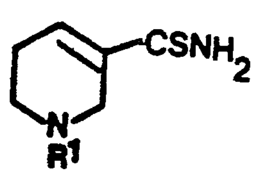

(II)

wherein $R^1$ has the meaning defined above, with a compound having the formula III

$R'-C(OCH_3)_2N(CH_3)_2$ (III)

wherein $R'$ has the meaning defined above to form a compound of the general formula I, wherein $R^3$ is

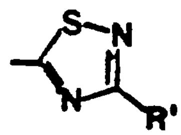

wherein $R'$ has the meaning defined above, or

b) alkylating a compound having the formula IV

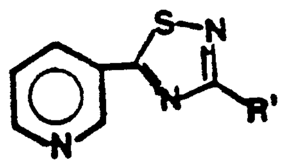

(IV)

with an alkyl halide and reducing the compound thus formed with hydride ions to form a compound having the formula V

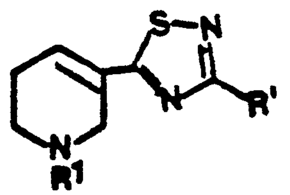

(V)

wherein $R^1$ has the meaning defined above.

The pharmacological properties of the compounds of the invention can be illustrated by determining their capability to inhibit the specific binding of $^3$H-Oxotremorine-M ($^3$H-Oxo).

$^3$H-Oxo labels muscarinic receptor in the CNS (with a preference for agonist domaines of the receptors. Three different sites are labelled by $^3$H-Oxo. These sites have affinity of 1.8, 20 and 3000 nM, respectively. Using the present experimental conditions only the high and medium affinity sites are determined.

The inhibitory effects of compounds on $^3$H-Oxo binding reflects the affinity for muscarinic acetylcholin receptors.

All preparations are performed at 0-4°C unless otherwise indicated. Fresh cortex (0.1-1 g) from male Wistar rats (150-250 g) is homogenized for 5-10 s in 10 ml 20 mM Hepes pH: 7.4, with an Ultra-Turrax homogenizer. The homogenizer is rinsed with 10 ml of buffer and the combined suspension centrifuged for 15 min at 40,000 $\times$ g. The pellet is washed three times with buffer. In each step the pellet is homogenized as before in 2$\times$10 ml of buffer and centrifuged for 10 min at 40,000 $\times$ g.

The final pellet is homogenized in 20 mM Hepes pH: 7.4 (100 ml per g of original tissue) and used for binding assay. Aliquots of 0.5 ml is added 25 $\mu$l of test solution and 25 $\mu$l of $^3$H-Oxotremorine (1.0 nM, final concentration) mixed and incubated for 30 min at 25°C. Non-specific binding is determined in triplicate using Arecholin (1 $\mu$g/ml, final concentration) as the test substance. After incubation samples are added 5 ml of ice-cold buffer and poured directly onto Whatman GF/C glass fiber filters under suction and immediately washed 2 times with 5 ml of ice-cold buffer. The amount of radioactivity on the filters are determined by conventional liquid scintillation counting. Specific binding is total binding minus non specific binding.

Test substances are dissolved in 10 ml water (if necessary heated on a steambath for less than 5 minutes) at a concentration of 2.2 mg/ml. 25-75% inhibition of specific binding must be obtained before calculation of $IC_{50}$.

The test value will be given as $IC_{50}$ (the concentration (ng/ml) of the test substance which inhibits the specific binding of $^3$H-Oxo by 50%).

$IC_{50}$ = (applied test susbtance concentration)

$$x \frac{1}{\left(\frac{C_o}{C_x} - 1\right)} \ ng/ml$$

where $C_o$ is specific binding in control assays and $C_x$ is the specific binding in the test assay. (The calculations assume normal mass-action kinetics).

The test results obtained by testing some compounds of the invention will appear from the following table:

TABLE 1

| Compound of invention | Inhibition in vitro oxo-binding (μg/ml) |
|---|---|
| Example 1 | 2.4 |
| Example 2 | 11 |
| Example 3 | 1.6 |

The compound of the invention, together with a conventional adjuvant, carrier, or diluent, and if desired in the form of a pharmaceutically-acceptable acid addition salt thereof, may be placed into the form of pharmaceutical compositions and unit dosages thereof, and in such form may be employed as solids, such as tablets or filled capsules, or liquids, such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective muscarinic cholinergic agonistic amount of the active ingredient commensurate with the intended daily dosage range to be employed. Tablets containing ten (10) milligrams of the active ingredient or, more broadly, one (1) to hundred (100) milligrams, per tablet, are accordingly suitable representative unit dosage forms.

The compounds of this invention can thus be used for the formulation of pharmaceutical preparations, e.g. for oral and parenteral administration to mammals including humans, in accordance with conventional methods of galenic pharmacy.

Conventional excipients are such pharmaceutically acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxiliary agents, emulsifiers, salt for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Ampoules are convenient unit dosage forms.

Tablets, dragees, or capsules having talc and/or a carbohydrate carrier or binder or the like, the carrier preferably being lactose and/or corn starch and/or potato starch, are particularly suitable for oral application. A syrup, elixir of the like can be used in cases where a sweetened vehicle can be employed.

Generally, the compounds of this invention are dispensed in unit form comprising 1-100 mg in a pharmaceutically acceptable carrier per unit dosage.

The dosage of the compounds according to this invention is 1-100 mg/day, preferably 10-70 mg/day, when administered to patients, e.g. humans, as a drug.

A typical tablet which may be prepared by conventional tabletting techniques contains:

| | | |
|---|---|---|
| Active compound | 5.0 | mg |
| Lactosum | 67.8 | mg Ph.Eur. |
| Avicel® | 31.4 | mg |
| Amberlite® IRP 88 | 1.0 | mg |
| Magnesii stearas | 0.25 | mg Ph.Eur. |

Due to the high muscarinic cholinergic receptor agonistic activity, the compounds of the invention are extremely useful in the treatment symptoms related to a reduction of the cognitive functions of the brain of mammals , when administered in an amount effective for stimulating the cognitive functions of the forebrain and hippocampus. The important stimulating activity of the compounds of the invention includes both activity against the patophysiological disease, Alzheimer's disease as well as against normal degeneration of brain function . The compounds of the invention may accordingly be administered to a subject, e.g., a living animal body, including a human, in need of stimulation of the cognitive functions of the forebrain and hippocampus, and if desired in the form of a pharmaceutically-acceptable acid addition salt thereof (such as the hydrobromide, hydrochloride, or sulfate, in any event prepared in the usual or conventional manner, e.g., evaporation to dryness of the free base in solution together with the acid), ordinarily concurrently, simultaneously, or together with a pharmaceutically-acceptable carrier or diluent, especially and prefe rably in the form of a pharmaceutical composition thereof, whether by oral, rectal, or parenteral (including subcutaneous) route, in an effective forebrain and hippocampus stimulating amount, and in any event an amount which is effective for improving the cognitive function of mammals due to their muscarinic cholinergic receptor agonistic activity. Suitable dosage ranges are 1-100 milligrams daily, 10-100 milligrams daily, and especially 30-70 milligrams daily, depending as usual upon the exact mode of administration, form in which administered, the indication toward which the administration is directed, the subject involved and the body weight of the subject involved, and the preference and experience of the physician or veterinarian in charge.

The invention will now be described in further detail with reference to the following examples:

## EXAMPLE 1

### a. 1,2,5,6-Tetrahydro-1-methyl-3-pyridinecarbothioamide

A solution of 1,2,5,6-tetrahydro-1-methyl-3-pyridinecarboxamide (4.2 g, 30.2 mmol) and Lawesson's reagent (6.1 g, 15.1 mmol) in HMPA (80 ml) was stirred at 80°C for 2.5 h. After cooling to room temperature, a saturated potassium carbonate solution (100 ml) was added to the reaction mixture. The water/HMPA solution was extracted with ether (3 x 500 ml) and the combined ether phases were dried, filtered and evaporated. The residue was chromatographed on silica gel using $CH_2Cl_2$:MeOH (9:1) as eluent. The tower spot was the wanted product, which could be isolated as a yellow solid in 410 mg yield. MS:$M^+$ = 156 (100%), 123 (90%).

### b. 1,2,5,6-Tetrahydro-1-methyl-3-(3-methyl-1,2,4-thiadiazol-5-yl)-pyridine oxalate

A solution of 1,2,5,6-tetrahydro-1-methyl-3-pyridinecarbothioamide (360 mg, 2.3 mmol) in N,N-dimethylacetamide dimethyl acetate (2.5 ml, 17 mmol) was stirred at room temperature for 3 h. The reaction mixture was evaporated in vacuo forming a black-brown oil. After dissolving the residue in EtOH (7 ml, 99.9%) and pyridine (0.18 ml) a solution of hydroxylamine-O-sulphonic acid (283 mg, 2.5 mmol) in MeOH (3 ml) was added. The reaction mixture was stirred at room temperature for 1 h and evaporated. The residue was dissolved in a saturated potassium carbonate solution (5 ml) and extracted with ether (5 x 20 ml). The combined organic phases were dried and evaporated. Chromatographic purification of the residue on silica gel (elution with $CH_2Cl_2$:MeOH (9:1) gave 130 mg of the wanted product. $^1$HNMR (60 $MH_2$, $CDCl_3$): 6.65 (1H,m), 3.35 (2H, pert. s), 2.60 (3H,s), 2.42 (3H,s), 2.8-2.2 (4H,m). Crystallization with oxalic acid from acetone gave 130 mg of the title compound. M.p. 175-177°C. GC/MS: $M^+$: 195 (100%).

## EXAMPLE 2

a. 3-(1,2,4-thiadiazol-5-yl)pyridine

A solution of thionicotinamide (10.0 g, 72.5 mmol) in N,N-dimethylformamide dimethylacetal (51 ml, 359 mmol) was stirred at 90°C for 3 h, followed by 16 h at room temperature. After evaporation, the brown oil residue was dissolved in ethanol (200 ml) and pyridine (5.7 ml). A solution of hydroxylamine-O-sulfonic acid (9.75 g, 86 mmol) in methanol (90 ml) was added at room temperature and the reaction mixture was stirred for 3 h. The reaction was evaporated, and the residue dissolved in water. The water phase was extracted with ether after addition of potassium carbonate to pH 10. The combined ether phases were dried and pyrified by column chromatography using ethyl acetate as eluent. Yield: 1.5 g. $M^+$: 163 (100%).

b. 1,2,5,6-Tetrahydro-1-methyl-3-(1,2,4-thiadiazol-5-yl)pyridine oxalate

To a solution of 3-(1,2,4-thiadiazol-5-yl)pyridine (1.5 g, 9.2 mmol) in acetone (50 ml) was added methyliodide (6 ml, 100 mmol). The reaction mixture was stirred at room temperature for 16 h and the precipitate collected by filtration, 1.98 g. The precipitate was dissolved in methanol (50 ml) and cooled to 0°C. Sodium borohydride (448 mg, 11.8 mmol) was added to the reaction micture. After 15 min. at 0°C the reaction was evaporated. The residue was dissolved in water and extracted with ether, dried and evaporated. Crystallization as the oxalate salt from acetone gave the title compound in 370 mg yield. M.p. 166-170°C dec. $M^+$: 181.

## EXAMPLE 3

a. Ethyl thionicotinate

A mixture of ethyl nicotinate (40 g, XXXX) and Lawesson's reagent (145 g, XXXX) in toluene (320 ml) was refluxed for 16 h. The reaction mixture was transferred to a $SiO_2$ column and eluated with toluene, yielding 9.0 g of pure title compound.

b. 3-(3-amino-1,2,4-thiadiazol-5-yl)pyridine

To a suspension of acetylguandine (5.45 g, 54 mmol) in dry tetrahydrofuran (120 ml) was added sodiumhydride (2.74 g oil suspension, 57 mmol) at 0°C. A solution of ethyl thionicotinate (9.0 g, 53.9 mmol) in tetrahydrofuran was added to the above mentioned suspension at room temperature. After the gas evolution was ended the reaction mixture wass filtered. Petroleum ether (11) was added to the filtrate causing spontaneous precipitation. The precipitate was dissolved in ethanol (90 ml) and acetic acid (2.95 ml). At 30°C a solution of $Br_2$ (3.2 ml) in chloroform (16 ml) was added dropwise. The reaction mixture was stirred for 30 min. at room temperature, filtered and evaporated. The residue was dissolved in aqueous potassium carbonate and extracted with methylene chloride. The combined organic phases were dried and evaporated. The title compound crystallized from ethyl acetate. Yield: 3.2 g. $M^+$: 178.

c. 3-(3-amino-1,2,4-thiadiazol-5-yl)-1,2,5,6-tetrahydro-1-methylpyridine oxalate

Methyl iodide (1.9 ml, 30 mmol) was added to a solution of 3-(3-amino-1,2,4-thiadiazol-5-yl)pyridine (1.8 g, 10.1 mmol) in acetone (80 ml). The reaction mixture was stirred at 45°C for 16 h, cooled to 0°C and the precipitate collected by filtration. Yield: 1.17 g. The precipitate was dissolved in methanol (100 ml) and water (25 ml). At 0°C sodium borohydride (278 mg, 7.3 mmol) was added to the solution. After stirring the reaction mixture for 15 min. water was added and extracted with ether. The combined ether phases were dried, evaporated and purified by column chromatography using ethyl acetate/methanol (2:1) as eluent.

Crystallization with oxalic acid from acetone gave the title compound in 350 mg yield. M.p. 194°C dec. $M^+$: 196.

## Claims

1. Azacyclic compounds having the general formula

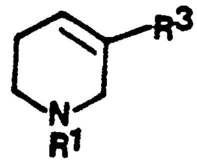

(I)

wherein
$R^1$ is H or $C_{1-6}$-alkyl, and

$R^3$ is 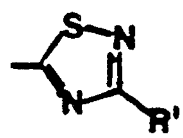

wherein $R'$ is hydrogen, $C_{1-6}$-alkyl, $C_{3-8}$-cycloalkyl, $C_{1-4}$-alkoxy, $C_{1-6}$-alkenyl, $C_{1-6}$-alkynyl, halogen or amino.

2. A compound of claim 1 selected from 1,2,5,6-tetrahydro-1-methyl-3-(3-methyl-1,2,4-thiadiazol-5-yl)-pyridine; 1,2,5,6-tetrahydro-1-methyl-3-(1,2,4-thiadiazol-5-yl)pyridine; 3-(3-amino-1,2,4-thiadiazol-5-yl)-1,2,5,6-tetrahydro-1-methyl)pyridine

3. A method of preparing a compound according to claim 1,
CHARACTERIZED in

a) reacting a reactive derivative of a compound having the formula II

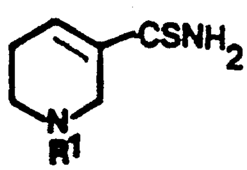

(II)

wherein $R^1$ has the meaning defined above, with a compound having the formula III

$R'-C(OCH_3)_2N(CH_3)_2$ (III)

wherein $R'$ has the meaning defined above to form a compound of the general formula I, wherein $R^3$ is

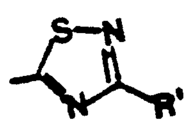

wherein $R'$ has the meaning defined above, or

b) alkylating a compound having the formula IV

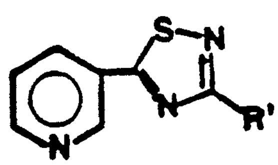

(IV)

with an alkyl halide and reducing the compound thus formed with hydride ions to form a compound having the formula V

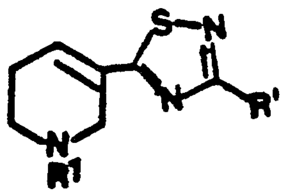

(V)

wherein $R^1$ has the meaning defined above.

4. A pharmaceutical composition suitable for use in stimulating the cognitive functions of the forebrain and hippocampus of mammals, including humans, and in treating Alzheimers disease, comprising an amount of a compound of claim 1 which is effective for the stimulation of the forebrain and hippocampus of mammals or treating Alzheimer's disease together with a pharmaceutically-acceptable carrier or diluent.

5. A pharmaceutical composition according to claim 4 wherein it is in the form of an oral dosage unit containing 1-100 mg of the active compound.

European Patent Office

# EUROPEAN SEARCH REPORT

Application number

EP 89112122.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| P,A | EP - A2 - 0 296 721 (LUNDBECK) * Claims 1,4,5; examples 74,78 * -- | 1,4,5 | C 07 D 417/04<br>A 61 K 31/44 |
| A | EP - A2 - 0 259 621 (FERROSAN) * Claims 1,4,5 * -- | 1,4,5 | |
| A | EP - A1 - 0 244 018 (AKZO) * Claims 1,8 * -- | 1,4,5 | |
| A | EP - A2 - 0 239 309 (MERCK) * Claims 1,8 * -- | 1,4,5 | |
| A | US - A - 4 650 805 (JAEN) * Claims 1,11 * ---- | 1,4,5 | |

TECHNICAL FIELDS SEARCHED (Int. Cl.4)

C 07 D 417/00

The present search report has been drawn up for all claims

| Place of search | Date of completion of the search | Examiner |
|---|---|---|
| VIENNA | 02-10-1989 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document